# EUROPEAN PATENT APPLICATION

(11) **EP 3 543 722 A1**
(43) Date of publication of application: **25.09.2019**
(21) Application number: 18162697.9
(22) Date of filing: 20.03.2018
(51) Int. Cl.: G01R 33/385, G01R 33/565, A61B 5/055, H03F 1/32

(54) **MAGNETIC RESONANCE IMAGING USING CORRECTED K-SPACE TRAJECTORIES CALCULATED FROM CURRENT SENSOR DATA**

(71) Applicant: Koninklijke Philips N.V., 5656 AE Eindhoven (NL)
(72) Inventor: LIPS, Oliver, 5656 AE Eindhoven (NL); BOERNERT, Peter, 5656 AE Eindhoven (NL); RAHMER, Jürgen Erwin, 5656 AE Eindhoven (NL); OVERWEG, Johannes Adrianus, 5656 AE Eindhoven (NL)
(74) Representative: de Haan, Poul Erik

(57) **Abstract**

The invention provides for a magnetic resonance imaging system (100, 300, 500) with a gradient coil system (110, 112, 113) that comprises a set of gradient coils (110) configured for generating a gradient, a gradient coil amplifier (112), and a current sensor system (113) configured for measuring current sensor data (146) descriptive of the electrical current supplied to each of the set of gradient coils. Execution of the machine executable instructions causes a processor to: control (200) the magnetic resonance imaging system with the pulse sequence commands (142) to acquire magnetic resonance imaging data; record (202) the current sensor data during the acquisition of the magnetic resonance imaging data; calculate (204) a corrected k-space trajectory (150) using the current sensor data and a gradient coil transfer function (148); and reconstruct (206) a corrected magnetic resonance image (152) using the magnetic resonance imaging data and the corrected k-space trajectory.

## Description

### FIELD OF THE INVENTION

The invention relates to magnetic resonance imaging, in particular to the gradient coil systems of magnetic resonance imaging systems.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field. During an MRI scan, Radio Frequency (RF) pulses generated by a transmitter coil cause perturbations to the local magnetic field, and RF signals emitted by the nuclear spins are detected by a receiver coil. These RF signals are recorded as magnetic resonance data and may be used to construct the MRI images. The transmitted RF field is referred to as the B1 field.
To differentiate different locations, spatially and temporally dependent gradient magentic fields are superimposed on an imaging zone. Varying the gradient magentic field enables spatial encoding of the RF signals emitted by the nuclear spins. The gradient magnetic fields in conjunction with the radio frequency pulses define paths in k-space along which the magentic resonacne data is sampled.

The journal article Spielman et al., "Spiral imaging on a small-bore system at 4.7T" Magn Reson Med. 1995, 34(4), pp. 580-5 discloses a spiral imaging technique where a constant voltage gradient waveform is used to reduce readout times as well as to minimize waveform distortion due to gradient amplifier nonlinearities. The gradients were measured under the same condition as was used for imaging experiments. The k-space trajectories were then obtained by integrating the gradient waveforms. A gridding reconstruction algorithm was then used to resample the data, and the image was then reconstructed using a 2DFT.

### SUMMARY OF THE INVENTION

The invention provides for a magnetic resonance imaging system, a computer program product, and a method in the independent claims. Embodiments are given in the dependent claims.

Embodiments of the invention may provide for improved image quality by measuring current sensor data during actual acquisition of the magnetic resonance imaging data. This has the advantage that the correct k-space trajectory can be calculated each time the magnetic resonance imaging data is acquired.

Further examples may refine this process further by maintaining a database or collection of gradient coil transfer functions. Different acquisition parameters such as the location of the subject support or the type of transmit / receive coil that is used can be used to choose a selected gradient coil transfer function. The conditions, as defined by the acquisition parameters, under which the magnetic resonance imaging data is acquired can affect eddy currents in the magnetic resonance imaging system and therefore affect the k-space trajectory.

In one aspect the invention provides for a magnetic resonance imaging system configured for acquiring magnetic resonance imaging data from an imaging zone. The magnetic resonance imaging system comprises a magnet for generating a main magnetic field within the imaging zone. The main magnetic field may also be referred to as the B0 magnetic field.

The magnetic resonance imaging system further comprises a gradient coil system for generating a gradient magnetic field within the imaging zone. The gradient coil system further comprises a set of gradient coils for generating the gradient magnetic field once applied with electrical current. Each of the set of gradient coils is configured for generating the gradient magnetic field along an axis. The gradient coil system comprises a gradient coil amplifier configured for supplying electrical current to each of the set of gradient coils. The gradient coil amplifier is therefore able to generate a gradient magnetic field along each of the individual axes or along a combination of them. The gradient coil system further comprises a current sensor system configured for measuring current sensor data descriptive of the electrical current supplied to each of the set of gradient coils by the gradient coil amplifier. The current sensor system may for example be incorporated into the gradient coil amplifier or it may be an external sensor.

The term current sensor system as used herein may be interpreted also comprising sensors for measuring the voltage also. For example, if the impedance of the gradient coil is known a voltage detector can be used to measure the voltage and calculate the current.

The magnetic resonance imaging system further comprises a memory containing machine-executable instructions. The memory further contains pulse sequence commands configured for controlling the magnetic resonance imaging system to acquire the magnetic resonance imaging data according to a magnetic resonance imaging protocol. The memory further contains a selected gradient coil transfer function configured for mapping the current sensor data to magnetic field components within the imaging zone. The selected gradient coil transfer function is a gradient coil transfer function. The term selected is to indicate a particular gradient coil transfer function. The magnetic field components comprise the gradient magnetic field. In some examples the magnetic field components may be identical with the gradient magnetic field. However, the transfer functions may contain terms for various field components. Typically, these field components are modeled as spherical harmonics and may commonly be recorded up to the third order of the spherical harmonics.

The magnetic resonance imaging system further comprises a processor for controlling the magnetic resonance imaging system. Execution of the machine-executable instructions causes the processor to control the magnetic resonance imaging system with the pulse sequence commands to acquire the magnetic resonance imaging data. Execution of the machine-executable instructions further causes the processor to record the current sensor data during the acquisition of the magnetic resonance imaging data. That is to say the current sensor data is acquired at the same time as the magnetic resonance imaging data. The acquisition of the magnetic resonance imaging data and the current sensor data may be correlated in time.

Execution of the machine-executable instructions further cause the processor to calculate a corrected k-space trajectory using the current sensor data and the selected gradient coil transfer function. There may be differences between the actual gradient magnetic field generated and that which is intended as is encoded in the pulse sequence commands. The corrected k-space trajectory is the actual trajectory along which the magnetic resonance imaging data is measured.

Execution of the machine-executable instructions further cause the processor to reconstruct a corrected magnetic resonance image using the magnetic resonance imaging data and the corrected k-space trajectory according to the magnetic resonance imaging protocol. It is possible that the corrected k-space trajectory deviates from the original k-space trajectory. The corrected k-space trajectory may be used in a variety of ways. For example, if the corrected k-space trajectory is non-Cartesian a so called gridding reconstruction algorithm may be used. This may enable a two-dimensional interpolation method that is effective with any k-space trajectory. This may for example involve re-sampling the magnetic resonance imaging data before the reconstruction of the corrected magnetic resonance image.

In another embodiment the memory further comprises a set of gradient coil transfer functions. Execution of the machine-executable instructions further causes the processor to receive one or more acquisition parameters descriptive of the magnetic resonance imaging protocol. Execution of the machine-executable instructions further cause the processor to choose the selected gradient coil transfer function using the acquisition parameters. This may be beneficial because there may be a number of conditions which affect the gradient coil transfer function. By have a set of gradient coil transfer functions it may be possible to select the most accurate gradient coil transfer function which may result in the highest quality of the corrected k-space trajectory.

In another embodiment the acquisition parameters comprise a subject height.

In another embodiment the acquisition parameters comprise the subject weight.

In another embodiment the acquisition parameters comprise a cryostat temperature of the magnet used for generating the main magnetic field.

In another embodiment the acquisition parameters comprise a cryostat state. The cryostat state may be for example data descriptive of a present operating condition of the magnet for generating the main magnetic field. The acquisition parameters may further comprise a subject support position of a subject support for the magnetic resonance imaging system. For example, the subject support may position the subject in the imaging zone during the acquisition of the magnetic resonance imaging data.

In another embodiment the acquisition parameters comprise a room temperature. The room temperature may be descriptive of a room temperature in the atmosphere surrounding the magnetic resonance imaging system and in particular the magnet.

In another embodiment the acquisition parameters comprise a gradient coil impedance of the set of gradient coils. Sensors for measuring the impedance of the set of gradient coils may be built into the gradient coil and/or the gradient coil amplifier.

In another embodiment the acquisition parameters comprise a gradient coil temperature. The gradient coil temperature may be descriptive of the temperature of the set of gradient coils at one or more locations. The gradient col temperature may be a single temperature measurement, a collection of temperature measurements, or an average temperature derived from the collection of temperature measurement.

In another embodiment the acquisition parameters comprise a gradient coil coolant temperature. The gradient coil coolant temperature may be descriptive of a coolant temperature of coolant used for cooling the set of gradient coils for the gradient coil system.

In another embodiment the acquisition parameters may comprise a magnetic resonance imaging protocol type of the magnetic resonance imaging protocol according to which the magnetic resonance imaging data is acquired and then an image is reconstructed.

In another embodiment the acquisition parameters further comprise a type of a receive coil. This may be descriptive of a type of receive coil currently being used in the magnetic resonance imaging system for the acquisition of the magnetic resonance imaging data.

In another embodiment execution of the machine-executable instructions further cause the processor to control the magnetic resonance imaging system to measure a current sensor signal and a gradient system response. The measurement of the gradient system response may for example be by executing pulse sequence commands that execute a routine for measuring the magnetic field within the imaging zone. This may be done with either a subject or a phantom within the imaging zone. Execution of the machine-executable instructions further causes the processor to calculate the selected gradient coil transfer function before acquiring the magnetic resonance imaging data. This may therefore be a precalibration step to correct the selected gradient coil transfer function.

In another embodiment the gradient amplifier is configured to receive a gradient control signal. This for example may be either an analogue or a digital control signal. A digital control signal may for example specify a waveform to be reproduced by the gradient amplifier. The memory further contains a selected gradient amplifier transfer function configured for mapping the gradient control signal to the electrical current supplied by the gradient amplifier. The pulse sequence commands are configured to provide the control signal during acquisition of the magnetic resonance imaging data. Execution of the machine-executable instructions further causes the processor to calculate a corrected control signal using the control signal and the selected gradient amplifier transfer function. The gradient amplifier is controlled with the corrected control signal during acquisition of the magnetic resonance imaging data. This example may be beneficial because the gradient amplifier may perform functionally as a low pass filter. This may enable a partial correction of the gradient control signal so that it more accurately generates the intended magnetic gradient field within the imaging zone.

In another embodiment execution of the machine-executable instructions further causes the processor to modify the selected gradient amplifier transfer function using the current sensor data recorded during acquisition of the magnetic resonance imaging data. As the gradient coil amplifier is operated the current sensor data can be used to measure if the output current is equal to the intended output current. If they differ this may be corrected at least partially by modifying the selected gradient amplifier transfer function.

In another embodiment the memory further comprises a set of gradient amplifier transfer functions. Execution of the machine-executable instructions further causes the processor to receive one or more system status parameters descriptive of a status of the magnetic resonance imaging system. Execution of the machine-executable instructions further causes the processor to choose the selected gradient amplifier transfer function from the set of gradient amplifier transfer functions using the one or more system status parameters. This embodiment may be beneficial because there may be a number of parameters upon which the operation of the gradient amplifier depends. This may enable a selection of a transfer function which enables more accurate use of the gradient amplifier.

In another embodiment the one or more system status parameters comprises a room temperature. The room temperature may for example be the temperature of the room in which the gradient amplifier and/or the magnet is in.

In another embodiment the one or more system parameters comprise a gradient coil impedance of the set of gradient coils. Sensors for measuring the impedance of the set of gradient coils may be built into the gradient coil and/or the gradient coil amplifier.

In another embodiment the one or more system status parameters comprises a gradient coil temperature. The gradient coil temperature may be temperature measurements at one or more locations in the set of gradient coils. The gradient col temperature may be a single temperature measurement, a collection of temperature measurements, or an average temperature derived from the collection of temperature measurement.

In another embodiment the one or more system status parameters comprise a prior use of the gradient amplifier. The prior use for example may be usage of the gradient amplifier within a predetermined amount of time above the time that the machine is currently being used. For example, the prior use may have a recording of how the gradient amplifier was used within the past 10 minutes, 30 minutes, 1 hour or several hours because this may have an effect on how the gradient amplifier functions.

In another embodiment the one or more system status parameters comprise a gradient amplifier temperature. The gradient amplifier temperature may for example be a temperature of the gradient amplifier.

In another embodiment the one or more system status parameters may comprise a magnetic resonance imaging protocol type. This may be beneficial because the magnetic resonance imaging protocol type may be used to differentiate different types of usage of the gradient amplifier.

In another embodiment execution of the machine-executable instructions further causes the processor to store the selected gradient amplifier transfer function after updating the set of gradient amplifier transfer functions. This embodiment may be beneficial because it may enable the continual improvement of the gradient amplifier transfer functions.

In another embodiment the selected gradient amplifier transfer function is selected from the set of gradient amplifier transfer functions using a machine learning algorithm. For example, the gradient amplifier transfer functions may be stored in a database with a variety of meta data which may comprise the system status parameters. The use of the machine learning algorithm may enable the selection of the most effective gradient amplifier transfer function from the set.

In another embodiment execution of the machine-executable instructions further causes the processor to train the machine learning algorithm with the system status parameters when storing the selected gradient amplifier transfer function in the set of gradient amplifier transfer functions. The machine learning algorithm could for example be a neural network, a convolution neural network, a statistical machine learning algorithm, an Isolation Forest algorithm, a k Nearest Neighbors algorithm, or a one-class support vector machine algorithm.

In another embodiment the corrected magnetic resonance image is at least partially reconstructed by re-gridding the magnetic resonance imaging data using the corrected k-space trajectory. This may entail a re-sampling process. This embodiment may be beneficial because it may enable straight forward reconstruction of the corrected magnetic resonance image.

In another aspect the invention provides for a method of operating a magnetic resonance imaging system configured for acquiring magnetic resonance imaging data from an imaging zone. The magnetic resonance imaging system comprises a magnet for generating a main magnetic field within the imaging zone. The magnetic resonance imaging system further comprises a gradient coil system for generating a gradient magnetic field within the imaging zone. The gradient coil system further comprises a set of gradient coils for generating the gradient magnetic field when supplied with electrical current. Each of the set of gradient coils is configured for generating the gradient magnetic field along an axis. The gradient coil system comprises a gradient coil amplifier configured for supplying the electrical current to each of the set of gradient coils. The gradient coil system further comprises a current sensor system. The current sensor system may be configured for measuring current sensor data descriptive of the electrical current supplied to each of the set of gradient coils by the gradient coil amplifier.

The method comprises controlling the magnetic resonance imaging system with the pulse sequence commands to acquire the magnetic resonance imaging data. The pulse sequence commands are configured for controlling the magnetic resonance imaging system to acquire the magnetic resonance imaging data according to a magnetic resonance imaging protocol. The method further comprises recording the current sensor data during the acquisition of the magnetic resonance imaging data. The method further comprises calculating a corrected k-space trajectory using the current sensor data and the selected gradient coil transfer function. The selected gradient coil transfer function is configured for mapping the current sensor data to magnetic field components within the imaging zone. The magnetic field components comprise the gradient magnetic field. The method further comprises reconstructing a corrected magnetic resonance image using the magnetic resonance imaging data and the corrected k-space trajectory according to the magnetic resonance imaging protocol.

In another aspect the invention provides for a computer program product comprising machine-executable instructions for execution by a processor controlling the magnetic resonance imaging system configured for acquiring magnetic resonance imaging data from an imaging zone. The magnetic resonance imaging system comprises a magnet for generating the main magnetic field within the imaging zone. The magnetic resonance imaging system further comprises a gradient coil system for generating a gradient magnetic field within the imaging zone. The gradient coil system further comprises a set of gradient coils for generating the gradient magnetic field when supplied with electrical current. Each of the set of gradient coils is configured for generating the gradient magnetic field along an axis. The gradient coil system comprises a gradient coil amplifier configured for supplying the electrical current to each of the set of gradient coils. The gradient coil system further comprises a current sensor system configured for measuring current sensor data descriptive of the electrical current supplied to each of the set of gradient coils by the gradient coil amplifier.

Execution of the machine-executable instructions causes the processor to control the magnetic resonance imaging system with pulse sequence commands to acquire the magnetic resonance imaging data. The pulse sequence commands are configured for controlling the magnetic resonance imaging system to acquire the magnetic resonance imaging data according to a magnetic resonance imaging protocol. Execution of the machine-executable instructions further causes the processor to record the current sensor data during the acquisition of the magnetic resonance imaging data. Execution of the machine-executable instructions further causes the processor to calculate a corrected k-space trajectory using the current sensor data and a selected gradient coil transfer function. The selected gradient coil transfer function is configured for mapping the current sensor data to magnetic field components within the imaging zone.

The magnetic field components comprise the gradient magnetic field. Execution of the machine-executable instructions further cause the processor to reconstruct a corrected magnetic resonance image using the magnetic resonance imaging data and the corrected k-space trajectory according to the magnetic resonance imaging protocol.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a 'circuit,' 'module' or 'system'. Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, random access memory (RAM), read only memory (ROM), an optical disk, a magneto-optical disk, and the register file of the processor. Examples of optical disks include compact disks (CD) and digital versatile disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising a 'processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as C or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the internet using an internet service provider).

Aspects of the present invention are described with reference to flowchart illustrations and / or block diagrams of methods, apparatus (systems) and computer program products according to embodiments of the invention. It is understood that each block or a portion of the blocks of the flowchart, illustrations, and / or block diagrams, can be implemented by computer program instructions in form of computer executable code when applicable. It is further understood that, when not mutually exclusive, combinations of blocks in different flowcharts, illustrations, and / or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions / acts specified in the flowchart and / or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function / act specified in the flowchart and / or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions / acts specified in the flowchart and / or block diagram block or blocks.

A 'user interface' as used herein is an interface which allows a user or operator to interact with a computer or computer system. A 'user interface' may also be referred to as a 'human interface device.' A user interface may provide information or data to the operator and / or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and / or control an external computing device and / or apparatus. A hardware interface may allow a processor to send control signals or instructions to an external computing device and / or apparatus. A hardware interface may also enable a processor to exchange data with an external computing device and / or apparatus. Examples of a hardware interface include, but are not limited to: a universal serial bus, IEEE 1394 port, parallel port, IEEE 1284 port, serial port, RS-232 port, IEEE 488 port, Bluetooth connection, wireless local area network connection, TCP / IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, a tactile electronic display, a Braille screen, a cathode ray tube (CRT), a storage tube, a bi-stable display, an electronic paper, a vector display, a flat panel display, a vacuum fluorescent display (VF), light-emitting diode (LED) displays, an electroluminescent display (ELD), plasma display panels (PDP), a liquid crystal display (LCD), organic light-emitting diode displays (OLED), a projector, and a head-mounted display.

Magnetic Resonance (MR) data is defined herein as being the recorded measurements of radio frequency signals emitted by atomic spins using the antenna of a magnetic resonance apparatus during an MRI scan. MR data is an example of medical image data. An MR image is defined herein as being the reconstructed two or three-dimensional visualization of anatomic data contained within the MRI data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a magnetic resonance imaging system;
Fig. 2 shows a flow chart which illustrates a method of using the magnetic resonance imaging system of Fig. 1;
Fig. 3 illustrates a further example of a magnetic resonance imaging system;
Fig. 4 shows a flow chart which illustrates a method of using the magnetic resonance imaging system of Fig. 4;
Fig. 5 illustrates a further example of a magnetic resonance imaging system;
Fig. 6 shows a flow chart which illustrates a method of using the magnetic resonance imaging system of Fig. 5;
Fig. 7 illustrates an example of electrical sensors used to measure the input and output of a gradient coil amplifier;
Fig. 8 illustrates the use of the gradient coil transfer function and the gradient amplifier transfer function;
Fig. 9 illustrates an example of a gradient coil transfer function;
Fig. 10 shows a block diagram which illustrates how the input to the gradient coil amplifier and the output of the gradient coil amplifier can be used to calculate a gradient amplifier transfer function;
Fig. 11 shows a sketch of the real component of a gradient amplifier transfer function; and
Fig. 12 shows a sketch of the phase of a gradient amplifier transfer function.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

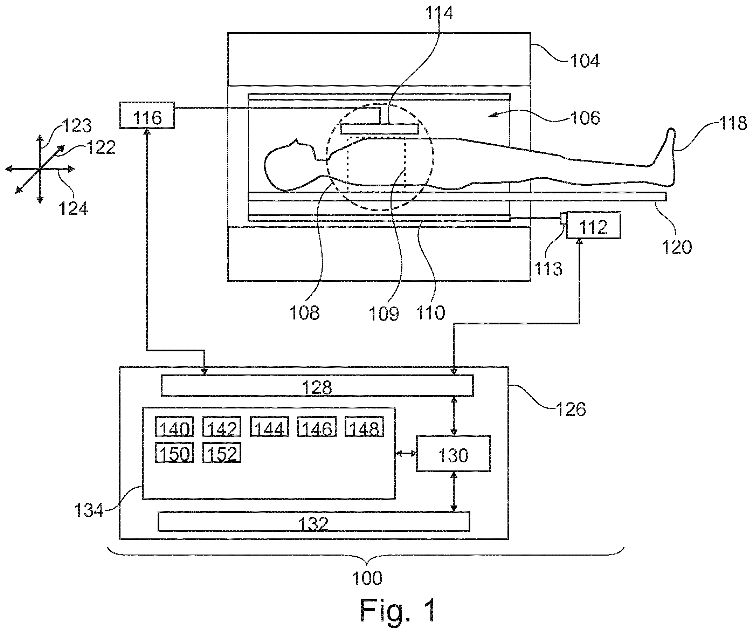

Fig. 1 illustrates an example of a magnetic resonance imaging system 100
with a magnet 104. The magnet 104 is a superconducting cylindrical type magnet with a bore 106 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 104 there is an imaging zone 108 where the magnetic field is strong and uniform enough to perform magnetic resonance imaging. A region of interest 109 is shown within the imaging zone 108. The magnetic resonance imaging data that is typically acquried for the region of interest. A subject 118 is shown as being supported by a subject support 120 such that at least a portion of the subject 118 is within the imaging zone 108 and the region of interest 109.

Within the bore 106 of the magnet there is also a set of set of gradient coils 110 which is used for acquisition of preliminary magnetic resonance imaging data to spatially encode magnetic spins within the imaging zone 108 of the magnet 104. The set of gradient coils 110 connected to a magnetic field gradient coil amplifier 112. The set of gradient coils 110 are intended to be representative. The set of gradient coils 110 contain three separate coils for spatially encoding in three orthogonal spatial directions. A magnetic field gradient power supply supplies current to the set of gradient coils. The current supplied to the set of gradient coils 110 is controlled as a function of time and may be ramped or pulsed.

The gradient coils 110 represent three separate sets of orthogonal gradient coils for generating a gradient magnetic field within the imaging zone 108. These are typically oriented as the axes 122, 123 and 124 show. The axis 124 is aligned with the axis of the magnet 104. This is typically referred to as the z-axis. 122 and 123 are the x and y-axes respectively. They are orthogonal to each other and also to the z-axis 124.

The magnetic field gradient coil amplifier 112 is configured for supplying current to each of the sets of gradient coils separately. The magnetic field gradient coil amplifier 112 is shown as having a current sensor system 113 for measuring the current supplied to each of the set of gradient coils 110. The current sensor system 113 could for example be part of the magnetic field gradient coil amplifier 112 or it could also be integrated into the set of gradient coils 110.

Adjacent to the imaging zone 108 is a radio-frequency coil 114 for manipulating the orientations of magnetic spins within the imaging zone 108 and for receiving radio transmissions from spins also within the imaging zone 108. The radio frequency antenna may contain multiple coil elements. The radio frequency antenna may also be referred to as a channel or antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise the transceiver 116 may also represent a separate transmitter and receivers. The radio-frequency coil 114 may also have multiple receive/transmit elements and the radio frequency transceiver 116 may have multiple receive/transmit channels. For example if a parallel imaging technique such as SENSE is performed, the radio-frequency could 114 will have multiple coil elements.

The transceiver 116 and the gradient controller 112 are shown as being connected to a hardware interface 128 of a computer system 126. The computer system further comprises a processor 130 that is in communication with the hardware system 128, a memory 134, and a user interface 132. The memory 134 may be any combination of memory which is accessible to the processor 130. This may include such things as main memory, cached memory, and also non-volatile memory such as flash RAM, hard drives, or other storage devices. In some examples the memory 134 may be considered to be a non-transitory computer-readable medium.

The memory 134 is shown as containing machine-executable instructions 140. The machine-executable instructions 140 enable the processor 130 to control the operation and function of the magnetic resonance imaging system 100. The machine-executable instructions 140 may also enable the processor 130 to perform various data analysis and calculation functions. The computer memory 134 is further shown as containing pulse sequence commands 142. The pulse sequence commands are configured for controlling the magnetic resonance imaging system 100 to acquire magnetic resonance imaging data.

The computer memory 134 is further shown as containing the magnetic resonance imaging data 144 that was acquired when the processor 130 executed the pulse sequence commands 142. The memory 134 is also shown as containing current sensor data 146. The current sensor data 146 was acquired at the same time as the magnetic resonance imaging data 144. The two 144, 146 are correlated in time so that it is possible to reconstruct a corrected k-space trajectory 150. The corrected k-space trajectory 150 is shown as being stored in the memory 134. The memory 134 is shown as containing a selected gradient coil transfer function 148. The selected gradient coil transfer function 148 is a mapping between the current sensor data 146 and magnetic field components that are generated within the imaging zone 108. The magnetic field components include the value of the gradient magnetic field generated by the gradient coils 110. The magnetic field components may also contain higher order terms.

The computer memory 134 is further shown as containing a corrected magnetic resonance image 152 that is reconstructed using the corrected k-space trajectory 150 and the magnetic resonance imaging data 144. In some examples there maybe resampled magnetic resonance imaging data 144 that is reconstructed as an intermediate step using the magnetic resonance imaging data and the corrected k-space trajectory 150.

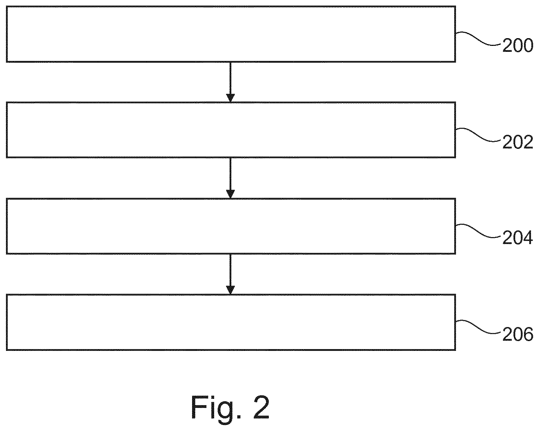

Fig. 2 shows a flowchart which illustrates a method of operating the magnetic resonance imaging system 100 of Fig. 1. First in step 200 the processor 130 controls the magnetic resonance imaging system 100 with the pulse sequence commands 142. This causes the magnetic resonance imaging system 100 to acquire the magnetic resonance imaging data 144. Step 202 is performed concurrently with step 200. In step 200 the processor 130 receives the current sensor data 146 from the current sensor system 113. Next in step 204 the processor 130 calculates a corrected k-space trajectory using the current sensor data 146 and the selected gradient coil transfer function 148. Finally, in step 206, the processor 130 reconstructs a corrected magnetic resonance image 152 using the magnetic resonance imaging data and the corrected k-space trajectory 150.

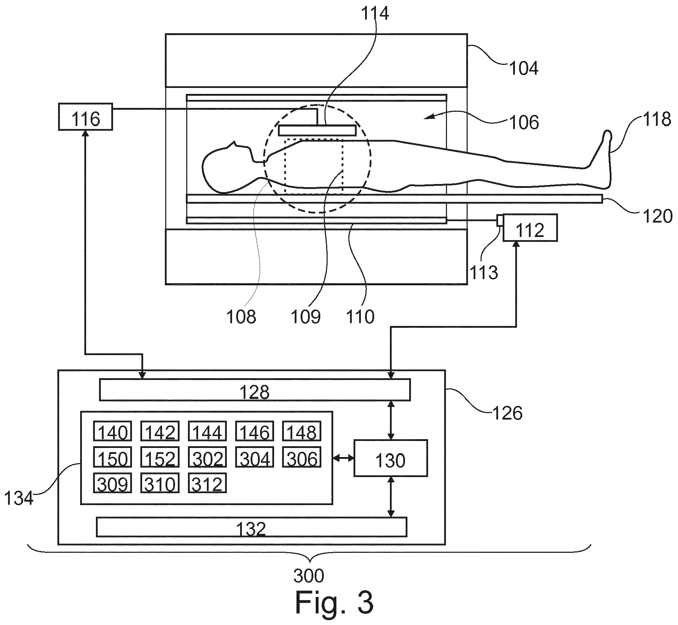

Fig. 3 illustrates a further example of a magnetic resonance imaging system 300. The magnetic resonance imaging system 300 of Fig. 3 is similar to that as is depicted in Fig. 1. The magnetic resonance imaging system 300 has several different additional elements or components within its memory 134. The memory 134 is shown as additionally containing a set of gradient coil transfer functions 302. The set of gradient coil transfer functions 302 is a number of gradient coil transfer functions which can be selected on the basis of the number of acquisition parameters. The selected gradient coil transfer function 148 in this example was selected from the set of gradient coil transfer functions 302. The acquisition parameters 304 are a number of parameters which describe the acquisition or conditions of the acquisition when the magnetic resonance imaging data 144 was acquired.

Examples of what the acquisition parameters could be could be the subject 118 height, a weight of the subject 118, a cryostat temperature of the magnet 104, a state or operational condition of the cryostat for the magnetic 104, a position of the subject support 120 within the bore of the magnet 106, a temperature of the room which contains the magnet 104, a gradient coil 110 coolant temperature, a gradient coil temperature, a gradient coil impedance, a type of the magnetic resonance imaging protocol used for implementing the pulse sequence commands 142, a type of the receive coil 114 or combinations thereof. Some of the various parameters above may have an effect on the gradient coil transfer function. By using the acquisition parameters to select one it may facilitate the selection of a correct gradient coil transfer function 148. For example, the memory 134 could contain a machine learning algorithm 306 that is used to select the selected gradient coil transfer function 148 from the set of gradient coil transfer functions 302 using the acquisition parameters 304. This could be implemented in any number of ways such as a neural net or deep learning algorithm and also an algorithm which measures the distance such as a nearest neighbors' algorithm could be used for the selection also.

As an alternative the selected gradient coil transfer function could also be measured before the acquisition of the magnetic resonance imaging data 144. For example, the memory 134 may contain magnetic field measuring pulse sequence commands 310 that contain pulse sequence commands which enable a magnetic resonance imaging protocol that can measure the magnetic field within the imaging zone 108. Execution of these pulse sequence commands 310 may enable the processor 130 to acquire magnetic field magnetic resonance imaging data 312 and the calibration current sensor data 308. From the magnetic field magnetic resonance imaging data 312 a gradient system response 314 may be calculated. The calibration current sensor data 308 and the gradient system response 314 may then be used to calculate the selected gradient coil transfer function 148.

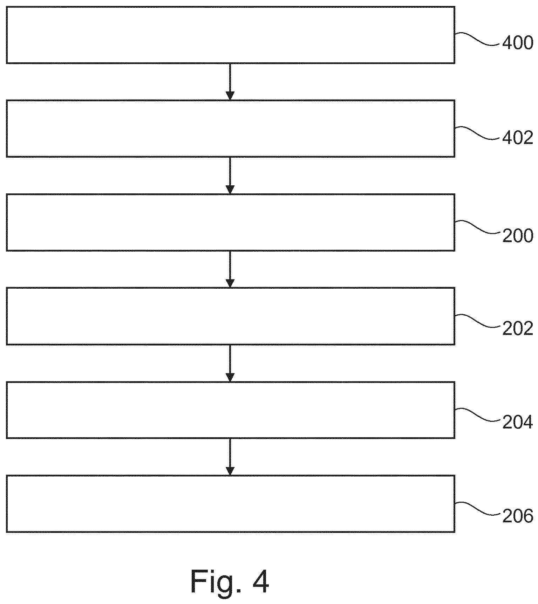

Fig. 4 shows a flowchart which illustrates a method of operating the magnetic resonance imaging system 300 of Fig. 3. First in step 400 the processor 130 receives one or more acquisition parameters 304. Next in step 402 the processor 130 chooses the selected gradient coil transfer function 148 using the acquisition parameters 304. The selected gradient coil transfer function 148 is selected from the set of gradient coil transfer functions 302. For example, the machine learning algorithm 306 may be used for this. After step 402 the method proceeds to step 200 of the method illustrated in Fig. 2.

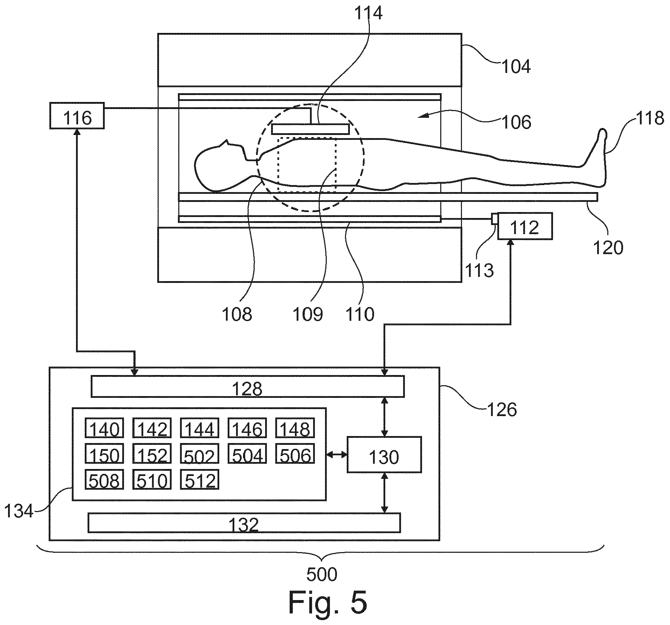

Fig. 5 illustrates a further example of a magnetic resonance imaging system 500. The magnetic resonance imaging system 500 is similar to that depicted in Fig. 1 with the addition of several additional elements or components in its memory 134. The features of the magnetic resonance imaging system which is illustrated in Figs. 1, 3 and 5 may be freely combined.

The memory 134 is further shown as containing a set of gradient amplifier transfer functions. A gradient amplifier transfer function 502 is a transfer function which interpolates an input into the gradient amplifier 112 to an output current. The memory 134 is further shown as containing a set of system status parameters 504. The system status parameters are parameters which may affect the operation of the gradient coil amplifier 112.

These for example may comprise a room temperature of the room in which the gradient coil amplifier 112 is placed in, it may contain a prior use of the gradient amplifier 112 for prior executions of pulse sequence commands, it may contain a temperature 112 measured within the gradient amplifier itself, a magnetic resonance imaging protocol type used for execution of the pulse sequence commands 142 and combinations thereof. The memory 134 is further shown as containing a selected gradient amplifier transfer function 506 that was selected from the set of selected gradient amplifier transfer functions 502 using the system status parameters 504. This may for example be done using a machine learning algorithm 508. The machine learning algorithm 508 may for example have a neural network, or another algorithm used to determine a closest neighbor based on the system status parameters. The set of gradient amplifier transfer functions may contain entries for the system status parameters 504 for each member of the set of gradient amplifier transfer functions. The machine learning algorithm 508 may then use the system status parameters to select the selected gradient amplifier transfer function 506.

The memory is further shown as containing a corrected control signal 510 which is used to control the gradient coil amplifier 112 during the execution of the pulse sequence commands 142. The selected gradient amplifier transfer function 506 is essentially a curve which measures the output of the amplifier 112 in relation to the input. Knowing this, the input can be corrected so that the output is more to the desired value. During the acquisition of the magnetic resonance imaging data 144 the current sensor data 146 is measured. The current sensor data 146 can be used to correct the selected gradient amplifier transfer function 506. The memory 134 shows a modified selected gradient amplifier transfer function 512 that was calculated using the current sensor data 146. The modified selected gradient amplifier transfer function 512 can for example be stored in the set of gradient amplifier transfer functions 502 along with the system status parameters 504. This can then be used to further train the machine learning algorithm 508.

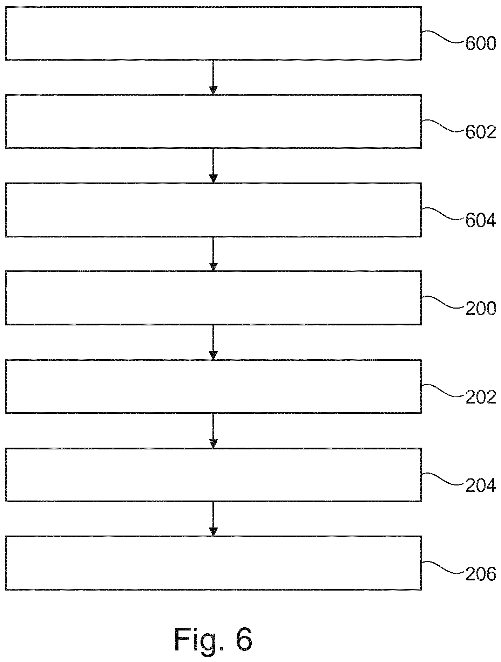

Fig. 6 shows a flowchart which illustrates a method of operating the magnetic resonance imaging system 500 of Fig. 5. First in step 600, the processor 130 receives one or more system status parameters system status parameters 504 descriptive of a status of the magnetic resonance imaging system 500. Next in step 602 the processor 130 chooses the selected gradient amplifier transfer function using the system status parameters 504 from the set of gradient amplifier transfer functions 502. This for example may be one using the machine learning algorithm 504. Then in step 604 the processor 130 calculates a corrected control signal 510 using the control signal from the pulse sequence commands 142 and the selected gradient amplifier transfer function 506. Next the method proceeds to step 200 as is illustrated in Fig. 2.

The gradient chain (the magnetic field gradient coil amplifier and the set of gradient coils) is an essential part of any MRI system. Its proper function is essential for the correct spatial encoding (of the magnetic resonance imaging data). Since real world hardware is prone to imperfections, any method to measure, characterize or predict deviations of the desired from the actual gradient trajectory (the corrected k-space trajectory) is of major interest. In this way the gradient waveform can either be pre-compensated or the measured data (magnetic resonance imaging data) can be accordingly corrected during reconstruction. Especially non-Cartesian trajectories are sensitive to trajectory deviations and thus would benefit from these improvements.

Since the deviations can evolve during scanning, e.g. caused by heating of the gradient coil (the set of gradient coils), characterizations of the gradient chain or output waveform "in realtime", i.e. simultaneously with the imaging may be beneficial. One way to accurately monitor the actual gradient waveform is the implementation of field probes, which quasi-continuously measure the MR-frequency at different locations. Although this is a very accurate method, it requires substantial additions of hardware, which can be very expensive. Furthermore, this additional hardware needs to be interfaced with the existing MR hard- and software, which adds further complexity to the entire MRI system.

Examples may use electrical sensors (current sensor system) to measure the currents and/or the voltages of the gradient coil and to deduce "on the fly" transfer functions of the gradient system (the gradient coil transfer functions and/or the gradient amplifier transfer functions), respectively their temporal changes. These sensors may already be present in the MR system and are used for the gradient amplifier control circuit, thus no additional hardware has to be implemented. It has turned out, that the accuracy of these measures is close to MR based methods in for characterization of the gradient chain. Additionally, the time signal of the current sensor can be directly used to determine the gradient trajectory applying a known (i.e. measured) transfer function from the current to the actual gradient (the selected gradient coil transfer function).

The actually applied gradient waveform is of major importance for the image quality, especially for non-Cartesian sampling. If the applied waveform significantly deviates from the desired one, substantial artefacts can occur. These deviations might be caused by:
- Frequency dependent amplitude variations and phase shifts (delays) occurring in the gradient chain (e.g. low pass behavior)
- Variations thereof due to temperature induced changes of the gradient chain/coil
- Non-linearities in elements of the gradient chain, e.g. the amplifier
- Etc.

Examples may provide for a means of identifying and characterizing one or more of the above effects. Using the measured output current of the gradient amplifier as a basis for trajectory calculation, all non-idealities occurring earlier in the gradient chain can be reduced or removed. In addition, examples may allow in general for a comparison of the measured gradient chain characteristics with the expected performance, thus enabling to identify potential hardware issues or defects (e.g. via the frequency dependent
impedance of the gradient coil).

A time dependent eddy current behavior may not necessarily directly measured by examples, however, this can be linked to the applied gradient currents and their history. Since these can be traced over time by examples, it is possible to apply predefined models that include also eddy current related effects.

Examples may use current and voltage sensors as well as the known gradient waveform input signal of the gradient amplifier as first order "field camera". Based on this one-dimensional information (Gx, Gy, Gz):
- The actual gradient system output and the resulting k-space trajectory can be estimated almost in real-time in a sense of a low budget field camera;
- Furthermore, this information can be used to determine corresponding transfer functions. These can be updated regularly and thus changes of the gradient chain can be detected. (Defining the transfer function in the frequency domain allows to characterize the gradient chain and possible changes thereof spectrally, thus providing additional information.)
- The ability to continuously monitor the gradient system under different load/duty cycle/working conditions allows to derive system specific generalized transfer functions using deep learning algorithms. This permits also to predict individual system performance learned from actual history to better steer reconstruction. The transfer functions, which can be regularly computed, are e.g. those relating the output currents to the desired input currents and those relating the actual output voltage to the output current (= the impedance). The continuous measurement of these functions allows to identify temperature drifts, changing delays, nonlinearities and potential defects.

In one example the time series of the current sensor is directly used to predict the actual gradient by applying a known transfer function between current and gradient (e.g. measured by an MR measurement)

Some examples use sensors at least for the gradient amplifier input signal, the output current and the output voltage for each axis of the gradient coils. These sensors may be connected to an ADC in order to allow for subsequent processing of the measured data. The current sensor may be of high accuracy, such sensors (e.g. current transducers) may be already used in some gradient amplifiers for the current control loop. Consequently, these sensors can also be used to implement examples and the additional hardware effort is minimal. Furthermore, depending of the hardware configuration the "demand input" information could already be present as digital information making explicit sampling obsolete.

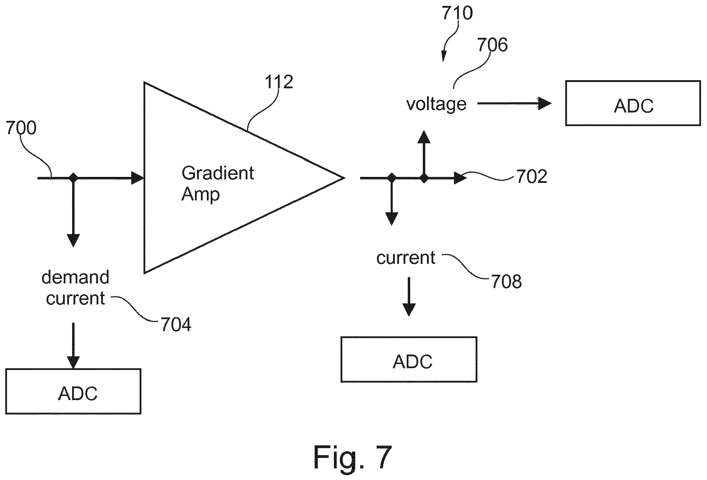

The same holds for voltage and input current sensors. An overview of the proposed setup is shown in Fig. 7. Fig. 7 illustrates an example of a current sensor system 710 which is an electrical sensor to measure and record input signals and output 702 of the gradient coil amplifier 112. In this example the input signal is an analogue signal which is then measured as the demand current 704 by an analogue-to-digital converter. In other examples the input into the gradient amplifier 112 could itself be a digital signal in which case the demand current 704 is already known. The current sensor system 710 may include a current 704 and optionally a voltage 706 measurement. Both the current 708 and the voltage 706 measurements could be measured by an analogue-to-digital converter. References herein to current sensor data may also include the digitized voltage 706 measurements.

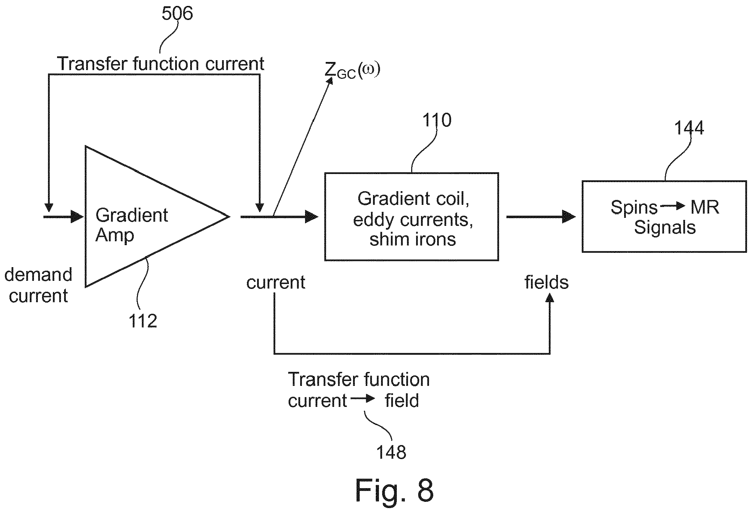

Fig. 8 shows a block diagram which illustrates the relationship of the selected gradient coil transfer function 148 to the selected gradient amplifier transfer function 506. The gradient amplifier 112 is shown as supplying current to the gradient coils 110. The transfer function for the gradient coil amplifier 112 relays a demand current or signal to resulting current. The current supplied to the gradient coil is then modeled using the selected gradient coil transfer function 148. This then results in the magnetic resonance imaging data 144 being acquired. The current sensor data can then be used to calculate a corrected k-space trajectory. Fig. 7 illustrates how to predicting the actual gradient trajectory from the measured currents applying a previously measured transfer function such as is shown in Fig. 8.

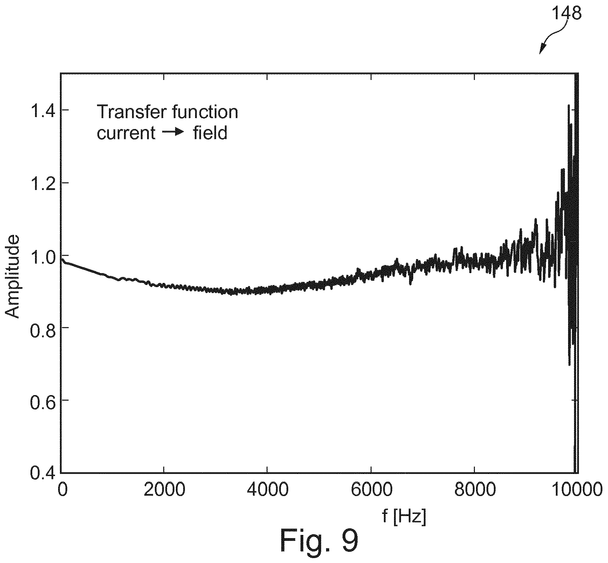

Fig. 9 illustrates an example of a gradient coil transfer function 148. This for example may relate the current supplied to one of the sets of gradient coils to the field within a particular voxel or location within the imaging zone.

The processing of the recorded (or available) signals performed in different ways. In a simple approach, the measured current is used in conjunction with a previously measured transfer (current to field gradient) to predict the actually applied gradient trajectory (i.e. based on the actually applied current). This approach is depicted in Fig. 8. The required transfer function can e.g. be deduced from a calibration measurement applying chirped or other gradients to determine a gradient impulse response function (GIRF). The calculation of the gradient trajectory can either be performed in frequency or time domain (convolution).

The determined gradient trajectory can finally be used for calculation of a highly accurate k-space trajectory during reconstruction.

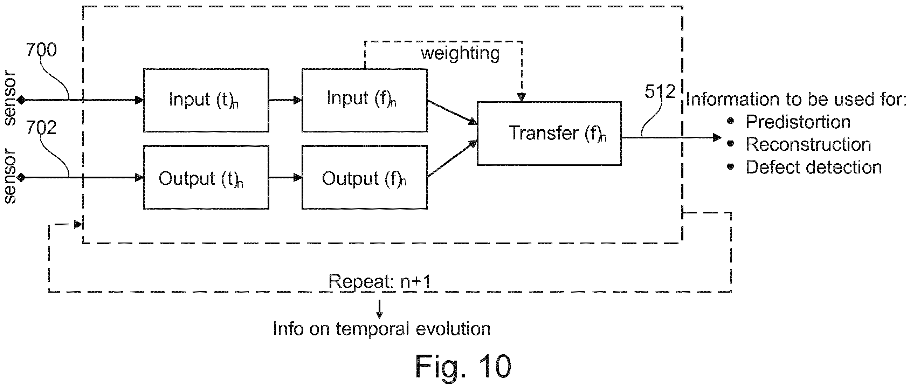

In another example, transfer functions (gradient amplifier transfer function) of the sensor signals in frequency domain are continuously calculated, as shown in Fig. 10. Fig. 10 shows a block diagram which illustrates how the input to the gradient coil amplifier 702 and the output of the gradient coil amplifier 702 can be used to calculate a gradient amplifier transfer function 512.

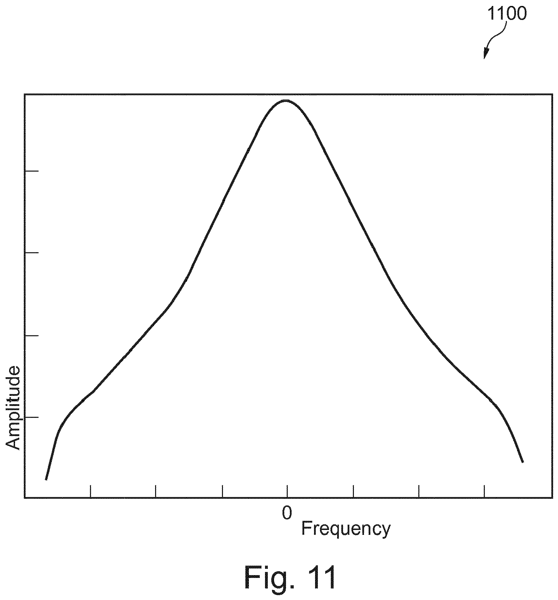

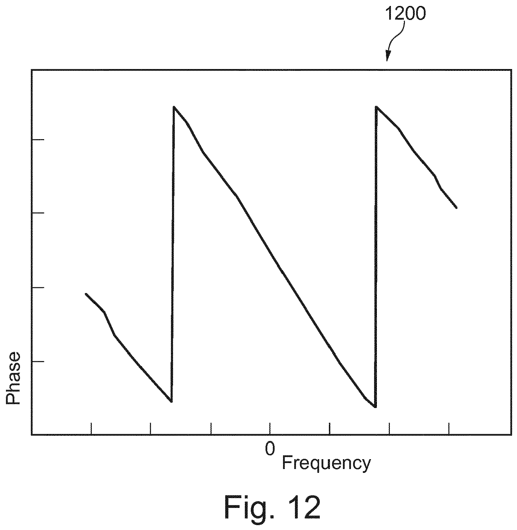

The transfer functions of interest are those relating input signal to output
current and those relating output voltage to output current (=impedance). Knowing these functions and also their evolution over time, the gradient trajectory can be deduced (as described above) but also temporal changes of the gradient chain can be identified. As an example a transfer function relating input signal to output current is shown in Figs 11 and 12. Figs. 11 and 12 show sketches of the real 1100 and the phase 1200 of a gradient amplifier transfer function. The units in these two figures are arbitrary. These functions shown in Figs. 11 and 12 and their temporal behavior provide substantial information on the performance of the gradient chain.

Similarly, the frequency dependent impedance provides important information on the gradient coil, the resistance can serve as a measure of gradient coil temperature and the resonance-like features in the inductance are due to mechanical resonances of the coil, which can provide insights in the proper function of the coil.

The frequency dependent impedance of the gradient coil as it can be determined using a transfer function approach. Heating can be observed as a gradual increase of the resistance near DC in the real part of the impedance. The inductance may show resonance-like structures at several frequencies, which are due to mechanical resonances. These features provide valuable information on the proper function of the gradient coil.

In a further application example, the sensor outputs are used, employing appropriate transfer function postprocessing to continuously monitor the gradient system under different load/duty-cycle/working conditions. Deep learning algorithms are employed to distil the key features of the transfer functions depending on the different system working conditions. This will result in system specific generalized transfer functions permitting to predict individual system performance learned from actual history to better steer reconstruction.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: magnetic resonance imaging system
- 104: magnet
- 106: bore of magnet
- 108: imaging zone
- 109: region of interest
- 110: set of gradient coils
- 112: magnetic field gradient coil amplifier
- 113: current sensor system
- 114: radio-frequency coil
- 116: transceiver
- 118: subject
- 120: subject support
- 122: x-axis
- 123: y-axis
- 124: z-axis
- 126: computer system
- 128: hardware interface
- 130: processor
- 132: user interface
- 134: computer memory
- 140: machine executable instructions
- 142: pulse sequence commands
- 144: magnetic resonance imaging data
- 146: current sensor data
- 148: selected gradient coil transfer function
- 150: corrected k-space trajectory
- 152: correccted magnetic resonance image
- 200: control the magnetic resonance imaging system with the pulse sequence commands to acquire the magnetic resonance imaging data
- 202: record the current sensor data during the acquisition of the magnetic resonance imaging data
- 204: calculate a corrected k-space trajectory using the current sensor data and the selected gradient coil transfer function
- 206: reconstruct a corrected magnetic resonance image using the magnetic resonance imaging data and the corrected k-space trajectory according to the magnetic resonance imaging protocol
- 300: magentic resonance imaging system
- 302: set of gradient coil transfer functions
- 304: acquisition parameters
- 306: machine learning algorithm
- 308: calibration current sensor data
- 310: magnetic field measureing pulse sequence commands
- 312: magnetic field magentic resonance data
- 314: gradient system response
- 400: receive one or more acquisition parameters descriptive of the magnetic resonance imaging protocol
- 402: choose the selected gradient coil transfer function using the acquisition parameters
- 500: magnetic resonance imaging system
- 502: set of gradient amplifier transfer functions
- 504: system status parameters
- 506: selected gradient amplifier transfer function
- 508: machine learning algorithm
- 510: corrected control singnal
- 512: modified selected graident amplifier transfer function
- 600: receive one or more system status parameters descriptive of a status of the magnetic resonance imaging system
- 602: choose the selected gradient amplifier transfer function from the set of gradient amplifier transfer functions using the one or more system status parameters
- 604: calculate a corrected control signal using the control signal and the selected gradient amplifier transfer function, wherein the gradient amplifier is controlled with the corrected control signal during acquisition of the magnetic resonance imaging data
- 700: input to gradient coil amplifier
- 702: output of gradient coil amplifier
- 704: demand current
- 706: voltage
- 708: current
- 710: current sensor system
- 1100: sketch of magnitude of gradient amplifier transfer function
- 1200: sketch of phase of gradient amplifier transfer function

## Claims

1. A magnetic resonance imaging system (100, 300, 500) configured for acquiring magnetic resonance imaging data (144) from an imaging zone (108), wherein the magnetic resonance imaging system comprises:
- a magnet (104) for generating a main magnetic field within the imaging zone;
- a gradient coil system (110, 112, 113) for generating a gradient magnetic field within the imaging zone, wherein the gradient coil system further comprises a set of gradient coils (110) for generating the gradient magnetic field when supplied with electrical current, wherein each of the set of gradient coils is configured for generating the gradient magnetic field along an axis (122, 123, 124), wherein the gradient coil system comprises a gradient coil amplifier (112) configured for supplying the electrical current to each of the set of gradient coils, and wherein the gradient coil system further comprises a current sensor system (113) configured for measuring current sensor data (146) descriptive of the electrical current supplied to each of the set of gradient coils by the gradient coil amplifier;
- a memory (134) containing machine executable instructions (140), wherein the memory further contains pulse sequence commands (142) configured for controlling the magnetic resonance imaging system to acquire the magnetic resonance imaging data according to a magnetic resonance imaging protocol, wherein the memory further contains a selected gradient coil transfer function (148) configured for mapping the current sensor data to magnetic field components within the imaging zone, wherein the magnetic field components comprise the gradient magnetic field;
- a processor (130) for controlling the magnetic resonance imaging system, wherein execution of the machine executable instructions causes the processor to:
- control (200) the magnetic resonance imaging system with the pulse sequence commands to acquire the magnetic resonance imaging data;
- record (202) the current sensor data during the acquisition of the magnetic resonance imaging data;
- calculate (204) a corrected k-space trajectory (150) using the current sensor data and the selected gradient coil transfer function; and
- reconstruct (206) a corrected magnetic resonance image (152) using the magnetic resonance imaging data and the corrected k-space trajectory according to the magnetic resonance imaging protocol.

2. The magnetic resonance imaging system of claim 1, wherein the memory further comprises a set of gradient coil transfer functions (302), wherein execution of the machine executable instructions further causes the processor to receive (400) one or more acquisition parameters (304) descriptive of the magnetic resonance imaging protocol, wherein execution of the machine executable instructions further causes the processor to choose (402) the selected gradient coil transfer function using the acquisition parameters.

3. The magnetic resonance imaging system of claim 2, wherein the acquisition parameters comprise any one of the following: the subject height, the subject weight, a cryostat temperature, a cryostat state, a subject support position, a room temperature, a gradient coil coolant temperature, a magnetic resonance imaging protocol type, a type of receive coil, a gradient coil temperature, a gradient coil impedance, and combinations thereof.

4. The magnetic resonance imaging system of any one of the preceding claims, wherein execution of the machine executable instructions further causes the processor to control the magnetic resonance imaging system to:
- measure calibration current sensor data using the current sensor system and a gradient coil system response using the magnetic resonance imaging system; and
- calculate the selected gradient coil transfer function using the calibration current sensor data and the gradient coil system response before acquiring the magnetic resonance imaging data.

5. The magnetic resonance imaging system of any one of the preceding claims, wherein the gradient amplifier is configured to receive a gradient control signal (700), wherein the memory further contains a selected gradient amplifier transfer function (506) configured for mapping the gradient control signal to the electrical current supplied by the gradient amplifier, wherein the pulse sequence commands are configured to provide the control signal during acquisition of the magnetic resonance imaging data, wherein execution of the machine executable instructions further cause the processor to calculate (604) a corrected control signal (510) using the control signal and the selected gradient amplifier transfer function, wherein the gradient amplifier is controlled with the corrected control signal during acquisition of the magnetic resonance imaging data.

6. The magnetic resonance imaging system of claim 5, wherein execution of the machine executable instructions further causes the processor to modify the selected gradient amplifier transfer function using the current sensor data recorded during acquisition of the magnetic resonance imaging data.

7. The magnetic resonance imaging system of claim 6, wherein the memory further comprises a set of gradient amplifier transfer functions (502), wherein execution of the machine executable instructions further causes the processor to receive (600) one or more system status parameters (504) descriptive of a status of the magnetic resonance imaging system, wherein execution of the machine executable instructions further causes the processor to choose (602) the selected gradient amplifier transfer function from the set of gradient amplifier transfer functions using the one or more system status parameters.

8. The magnetic resonance imaging system of claim 7, wherein the one or more system status parameters comprise any one of the following: a room temperature, a prior use of the gradient amplifier, a gradient amplifier temperature, a magnetic resonance imaging protocol type, a gradient coil temperature, a gradient coil impedance, and combinations thereof.

9. The magnetic resonance imaging system of claim 7 or 8, wherein execution of the machine executable instructions further causes the processor to store the selected gradient amplifier transfer function after updating in the set of gradient amplifier transfer functions.

10. The magnetic resonance imaging system of claim 9, wherein the selected gradient amplifier transfer function is selected from the set of gradient amplifier transfer functions using a machine learning algorithm (508).

11. The magnetic resonance imaging system of claim 10, wherein execution of the machine executable instructions further causes the processor to train the machine learning algorithm with the system status parameters when storing the selected gradient amplifier transfer function in the set of gradient amplifier transfer functions.

12. The magnetic resonance imaging system of any one of the preceding claims, wherein the corrected magnetic resonance imaging is at least partially reconstructed by regridding the magnetic resonance imaging data using the corrected k-space trajectory.

13. A method of operating a magnetic resonance imaging system (100, 300, 500) configured for acquiring magnetic resonance imaging data (144) from an imaging zone (108), wherein the magnetic resonance imaging system comprises a magnet (104) for generating a main magnetic field within the imaging zone, wherein the magnetic resonance imaging system further comprises a gradient coil system (110, 112, 113) for generating a gradient magnetic field within the imaging zone, wherein the gradient coil system further comprises a set of gradient coils (110) for generating the gradient magnetic field when supplied with electrical current, wherein each of the set of gradient coils is configured for generating the gradient magnetic field along an axis (122, 123, 124), wherein the gradient coil system comprises a gradient coil amplifier (112) configured for supplying the electrical current to each of the set of gradient coils, and wherein the gradient coil system further comprises a current sensor system (113) configured for measuring current sensor data descriptive of the electrical current supplied to each of the set of gradient coils by the gradient coil amplifier, wherein the method comprises:
- controlling (200) the magnetic resonance imaging system with the pulse sequence commands to acquire the magnetic resonance imaging data according to a magnetic resonance imaging protocol;
- recording (202) the current sensor data during the acquisition of the magnetic resonance imaging data;
- calculating (204) a corrected k-space trajectory (150) using the current sensor data and a selected gradient coil transfer function (148), wherein the selected gradient coil transfer function is configured for mapping the current sensor data to magnetic field components within the imaging zone, wherein the magnetic field components comprise the gradient magnetic field; and
- reconstructing (206) a corrected magnetic resonance image (152) using the magnetic resonance imaging data and the corrected k-space trajectory according to the magnetic resonance imaging protocol.

14. A computer program product comprising machine executable instructions (140) for execution by a processor (130) controlling a magnetic resonance imaging system (100, 300, 500) configured for acquiring magnetic resonance imaging data (144) from an imaging zone (108), wherein the magnetic resonance imaging system comprise a magnet (104) for generating a main magnetic field within the imaging zone, wherein the magnetic resonance imaging system further comprise a gradient coil system (110, 112, 113) for generating a gradient magnetic field within the imaging zone, wherein the gradient coil system further comprises a set of gradient coils (110) for generating the gradient magnetic field when supplied with electrical current, wherein each of the set of gradient coils is configured for generating the gradient magnetic field along an axis (122, 123, 124), wherein the gradient coil system comprises a gradient coil amplifier (112) configured for supplying the electrical current to each of the set of gradient coils, and wherein the gradient coil system further comprises a current sensor system (113) configured for measuring current sensor data descriptive of the electrical current supplied to each of the set of gradient coils by the gradient coil amplifier, wherein execution of the machine executable instructions causes the processor to:
- control (200) the magnetic resonance imaging system with pulse sequence commands (142) to acquire the magnetic resonance imaging data according to a magnetic resonance imaging protocol;
- record (202) the current sensor data during the acquisition of the magnetic resonance imaging data;
- calculate (204) a corrected k-space trajectory (150) using the current sensor data and a selected gradient coil transfer function, wherein the selected gradient coil transfer function is configured for mapping the current sensor data to magnetic field components within the imaging zone, wherein the magnetic field components comprise the gradient magnetic field; and
- reconstruct (206) a corrected magnetic resonance image (152) using the magnetic resonance imaging data and the corrected k-space trajectory according to the magnetic resonance imaging protocol.
